(19) Europäisches Patentamt
European Patent Office
Office européen des brevets

(11) EP 4 640 809 A1

(12) **EUROPEAN PATENT APPLICATION**

(43) Date of publication:
**29.10.2025 Bulletin 2025/44**

(21) Application number: **24222297.4**

(22) Date of filing: **20.12.2024**

(51) International Patent Classification (IPC):
***C11D 11/04*** *(2006.01)* ***C07C 303/24*** *(2006.01)*
***C11D 1/37*** *(2006.01)* *C11D 1/12 (2006.01)*
*C11D 1/14 (2006.01)* *C11D 1/29 (2006.01)*

(52) Cooperative Patent Classification (CPC):
(C-Sets available)
**C11D 11/04; C07C 303/24; C11D 1/37;** C11D 1/12;
C11D 1/146; C11D 1/29 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **23.04.2024 EP 24171777**

(71) Applicant: **The Procter & Gamble Company**
**Cincinnati, OH 45202 (US)**

(72) Inventors:
- **ARREDONDO, Victor Manuel**
**Cincinnati, 45202 (US)**
- **BILLIAUW, Jan Julien Marie-Louise**
**1853 Strombeek-Bever (BE)**
- **STERGIOPOULOU, Natalia**
**1853 Strombeek-Bever (BE)**
- **VINSON, Phillip Kyle**
**Cincinnati, 45202 (US)**

(74) Representative: **P&G Patent Belgium UK**
**N.V. Procter & Gamble Services Company S.A.**
**Temselaan 100**
**1853 Strombeek-Bever (BE)**

(54) **LIQUID HAND DISHWASHING COMPOSITION**

(57) The need for a liquid hand dishwashing composition which comprises surfactant which is derived from more renewable sources, while still providing good performance, especially suds-mileage in the presence of greasy-particulate soils and hard water, and grease cleaning, is met by formulating the liquid hand dishwashing composition using an alkyl alkanolamide sulfate anionic surfactant in combination with an alkyl sulfate anionic surfactant.

EP 4 640 809 A1

(52) Cooperative Patent Classification (CPC): (Cont.)

C-Sets
**C07C 303/24, C07C 305/06**

## Description

### FIELD OF THE INVENTION

The invention relates to liquid hand dishwashing detergent compositions, which provide good sudsing, viscosity, and stability, while still having high biodegradability and a high level of renewable components.

### BACKGROUND OF THE INVENTION

Hand dishwashing cleaning compositions have typically been formulated using alkyl ethoxylated sulfate surfactants as the principal anionic surfactant, since they provide good grease removal, suds mileage in the presence of suspended greasy-particulate soil, and good low temperature stability. Grease removal and suds mileage, in particular, are seen as indicators of good, long-lasting, performance.

During manual dishwashing, the user typically relies on the level of suds to indicate the remaining cleaning efficacy of the diluted detergent composition. A high suds volume and/or stable, long-lasting suds longevity (*i.e.*, mileage) indicates to the user that sufficient active ingredients (*e.g.*, surfactants) remain, in order to perform the desired cleaning. Poor suds longevity typically leads to the user dosing additional detergent composition even when cleaning efficacy remains. Moreover, as soil is lifted off from plates and dishes, the soil is emulsified and/or suspended into the wash liquor and further suppresses sudsing, even when there is sufficient surfactant present for further grease-cleaning. Poor suds-longevity in the presence of soil is further exacerbated when hard water is used.

There is also an increasing desire for detergent compositions which have improved biodegradability, and which are derived from renewable sources. Hand dishwashing cleaning compositions have typically been formulated using alkyl ethoxylated sulfate surfactants as the principal anionic surfactant. However, processes to make such alkyl ether sulfate anionic surfactants may result in trace residual amounts of 1,4-dioxane by-product being present. The amount of 1,4-dioxane by-product within alkoxylated especially ethoxylated alkyl sulfates can be reduced. Based on recent advances in technology, a further reduction of 1,4-dioxane by-product can be achieved by subsequent stripping, distillation, evaporation, centrifugation, microwave irradiation, molecular sieving or catalytic or enzymatic degradation steps.

An alternative is to use alkyl sulfate anionic surfactants which comprise only low levels of ethoxylation, or even being free of ethoxylation. However, formulating with such alkyl sulfate anionic surfactants, having little or no ethoxylation, has lead to reduced performance of the hand dishwashing detergent composition, especially in respect of suds longevity in the presence of greasy-particulate soil, and reduced physical stability at lower temperatures.

As such, there remains a need for liquid hand dishwashing compositions which comprises surfactant which is derived from more renewable sources, while still providing good performance, especially suds-mileage in the presence of greasy-particulate soils and hard water, and grease cleaning.

EP0780367A relates to an anionic detergent mixture obtained by sulphating a mixture of fatty acid alkanol amides and fatty alcohol polyalkylene glycols together in a weight ratio of 5:95 to 70:30 and subsequently neutralising with an aqueous base, as well as the process of co-sulphating the mixture together and neutralising the mixture with an aqueous base. US4116986A relates to a process for sulfating fatty alkanolamides prepared from the reaction of fatty acids and esters with low molecular weight alkanolamines with the addition of about 5 to 15% by weight of a low molecular weight alcohol to the fatty alkanolamide and consulfating with a sulfating agent. JPH10330783A relates to a detergent composition which contains at least one amidosulfuric ester surfactant. KR20120060485A relates to a liquid detergent composition comprising an alkyl alkanolamide sulfate anionic surfactant. US2353081A relates to sulphated monoethanolamides and process for the preparation thereof. US2843550A relates to detergent compositions consisting essentially of alkali metals of sulfated C2-C3 alkylolamides of C14-C18 hydrogenated tallow fatty acids, alkali metal C9-C18 monoalkyl benzene sulfonate detergent, the former being present in an amount, by weight, of about 15% to 50% and the latter, 50% to 85%, by weight, of the total of the two, and as a foam-improving agent\, about 5% to 70%, by weight, based on the sulfated alkylolamide salt, or a normal, saturated C10-C18 aliphatic alcohol. EP4253510A relates to a liquid hand dishwashing detergent composition comprising a higher fraction of components derived from natural, renewable sources, ideally also having improved biodegradability, and still providing good sudsing, grease removal, and low temperature stability, while not substantially changing the viscosity profile, the composition comprising a surfactant system which comprises anionic surfactant, the anionic surfactant comprising a combination of alkyl sulfate anionic surfactant and acyl taurate anionic surfactant. The synthesis of sulfated alkanolamides is described in "Synthesis and properties of sulfated alkanolamides" Journal of the American Oil Chemists' Society volume 47, pages91-93 (1970), and "Synthesis and Properties of N-Alkyl Amide Sulfates" Langmuir 1999, 15, 20, 6664-6670.

### SUMMARY OF THE INVENTION

The present invention relates to a liquid hand dishwashing detergent composition comprising from 5.0% to 50%

by weight of the liquid hand dishwashing detergent composition of a surfactant system, wherein the surfactant system comprises: anionic surfactant, wherein the anionic surfactant comprises: an alkyl alkanolamide sulfate anionic surfactant, wherein the alkyl alkanolamide sulfate anionic surfactant has the formula:

R(CO)NXR'O-SO3- M+ (I),

wherein: R is an alkyl chain comprising a number average of from 7 to 17 carbon atoms; R' is an alkyl chain comprising a number average of from 1 to 3 carbon atoms; X is H or C1 to C3 alkyl; and M+ is a counterion; and alkyl sulfate anionic surfactant, wherein the alkyl sulfate anionic surfactant has an average degree of alkoxylation of less than 0.1.

The present invention further relates to a process for making a concentrated surfactant blend comprising the alkyl alkanolamide sulfate anionic surfactant having the formula:

R(CO)NXR'O-SO3- M+ (I),

wherein the process comprises the following steps: providing a nonionic stream, wherein the nonionic stream comprises at least one alkyl alkanolamide of formula (II):

R(CO)NXR'OH (II);

a sulfation step, during which the at least one alkyl alkanolamide of formula (II) in the nonionic stream is sulfated to form a sulfuric acid stream comprising at least one alkyl alkanolamide sulfuric acid; providing a neutralising stream comprising at least one neutralising agent; a neutralisation step, during which the sulfuric acid stream and the neutralising stream are combined, in order to neutralise the at least one alkyl alkanolamide sulfuric acid.

## DETAILED DESCRIPTION OF THE INVENTION

Formulating the liquid cleaning composition as described herein, results in a hand dishwashing detergent composition having good suds mileage in the presence of greasy-particulate soils, and good grease removal while also providing a composition which is derived from more renewable sources. Greasy-particulate soils comprise a blend of grease or oil and particulates such as carbohydrates and the like.

As used herein, articles such as "a" and "an" when used in a claim, are understood to mean one or more of what is claimed or described.

The term "comprising" as used herein means that steps and ingredients other than those specifically mentioned can be added. This term encompasses the terms "consisting of" and "consisting essentially of." The compositions of the present invention can comprise, consist of, and consist essentially of the essential elements and limitations of the invention described herein, as well as any of the additional or optional ingredients, components, steps, or limitations described herein.

The term "dishware" as used herein includes cookware and tableware made from, by non-limiting examples, ceramic, china, metal, glass, plastic (*e.g.*, polyethylene, polypropylene, polystyrene, etc.) and wood.

The term "grease" or "greasy" as used herein means materials comprising at least in part (*i.e.*, at least 0.5 wt% by weight of the grease in the material) saturated and unsaturated fats and oils, preferably oils and fats derived from animal sources such as beef, pig and/or chicken.

The terms "include", "includes" and "including" are meant to be non-limiting.

The term "particulate soils" as used herein means inorganic and especially organic, solid soil particles, especially food particles, such as for non-limiting examples: finely divided elemental carbon, baked grease particle, and meat particles.

The term "sudsing profile" as used herein refers to the properties of a cleaning composition relating to suds character during the dishwashing process. The term "sudsing profile" of a cleaning composition includes initial suds volume generated upon dissolving and agitation, typically manual agitation, of the cleaning composition in the aqueous washing solution, and the retention of the suds during the dishwashing process. Preferably, hand dishwashing cleaning compositions characterized as having "good sudsing profile" tend to have high initial suds volume and/or sustained suds volume, particularly during a substantial portion of or for the entire manual dishwashing process. This is important as the consumer uses high suds as an indicator that enough cleaning composition has been dosed. Moreover, the consumer also uses the sustained suds volume as an indicator that enough active cleaning ingredients (e.g., surfactants) are present, even towards the end of the dishwashing process. The consumer usually renews the washing solution when the sudsing subsides. Thus, a low sudsing cleaning composition will tend to be replaced by the consumer more frequently than is necessary because of the low sudsing level.

It is understood that the test methods that are disclosed in the Test Methods Section of the present application

must be used to determine the respective values of the parameters of Applicants' inventions as described and claimed herein.

All percentages are by weight of the total composition, as evident by the context, unless specifically stated otherwise. All ratios are weight ratios, unless specifically stated otherwise, and all measurements are made at 25°C, unless otherwise designated.

Liquid cleaning composition

The cleaning composition is a liquid cleaning composition, preferably a liquid hand dishwashing cleaning composition, and hence is in liquid form. The liquid cleaning composition is preferably an aqueous cleaning composition. As such, the composition can comprise from 50% to 85%, preferably from 50% to 75%, by weight of the total composition of water.

The liquid cleaning composition has a pH of 7.0 or greater, or a pH of from 7.0 to 12.0, preferably from 7.5 to 11.0, more preferably from 8.0 to 10.0, measured as a 10% aqueous solution in demineralized water at 20 degrees °C.

The liquid cleaning composition of the present invention can be Newtonian or non-Newtonian, preferably Newtonian. Preferably, the composition has a viscosity of from 10 mPa·s to 10,000 mPa·s, preferably from 100 mPa·s to 5,000 mPa·s, more preferably from 300 mPa·s to 2,000 mPa·s, or most preferably from 500 mPa·s to 1,500 mPa·s, alternatively combinations thereof.

The compositions of the present invention may comprise renewable components and exhibit good performance, such as cleaning and suds mileage. The compositions disclosed herein may comprise from 20% or from 40% or from 50%, to 60% or 80% or even to 100% by weight of the compositions of renewable components. The compositions disclosed herein may be at least partially or fully bio-based. As such, the composition can comprise a bio-based carbon content of from 50% to 100%, preferably from 75% to 100%, most preferably from 80% to 100%, most preferably about 90% to about 100% by weight of the composition. By bio-based, it is meant that the material is derived from substances derived from living organisms such as farmed plants, rather than, for example, coal-derived or petroleum-derived. The percent bio-based carbon content can be calculated as the "percent Modern Carbon (pMC)" as derived using the methodology of ASTM D6866-16. The compositions of the present disclosure may be substantially free of petroleum-derived solvents. The compositions of the present disclosure may be substantially free of surfactants or even polymers derived from petroleum-derived alcohols.

Surfactant System

The liquid cleaning composition comprises from 5.0% to 50%, preferably from 6.0% to 40%, most preferably from 15% to 35%, by weight of the total composition of a surfactant system.

Anionic surfactant

The surfactant system comprises anionic surfactant. The surfactant system can comprise at least 40%, preferably from 60% to 90%, more preferably from 65% to 85% by weight of the surfactant system of the anionic surfactant. The surfactant system is preferably free of fatty acid or salt thereof, since such fatty acids impede the generation of suds.

Alkyl alkanolamide sulfate anionic surfactant

The surfactant system comprises an anionic surfactant, wherein the anionic surfactant comprises alkyl alkanolamide sulfate anionic surfactant. Preferably the alkyl alkanolamide sulfate anionic surfactant is present at a level of from 5.0 % to 50 % preferably from 10 % to 40 %, more preferably from 15 % to 30 % by weight of the anionic surfactant system.

The alkyl alkanolamide sulfate anionic surfactant can be present in a range of from 1.0 % to 25 % preferably from 3.0 % to 15 %, more preferably from 5.0 % to 10 % by weight of the composition.

The alkyl alkanolamide sulfate anionic surfactant of use in the present invention has the formula:

$$R(CO)NXR'O\text{-}SO_3^- M^+ \qquad (I),$$

wherein:

R is an alkyl chain comprising a number average of from 7 to 17 carbon atoms, preferably R is an alkyl chain comprising a number average of from 9 to 13, more preferably from 11 to 13 carbon atoms, and most preferably R is a blend of C11 and C13 alkyl chains;

R' is an alkyl chain comprising a number average of from 1 to 3 carbon atoms, preferably R' is an alkyl chain comprising a number average of from 2 to 3 carbon atoms, more preferably wherein R' is selected from the group consisting of: $-(CH_2)_2-$, $-CH_2CH(CH_3)-$, $-CH(CH_3)CH_2-$;

X is H or C1 to C3 alkyl, preferably X is H or C1 alkyl, more preferably H or methyl;

$M^+$ is a counterion, preferably $M^+$ is an alkali metal counterion or ammonium, ethanolamine, or isopropanolamine, more preferably $Na^+$, or $K^+$, $Mg^{2+}$, or ethanolamine, most preferably $Na^+$.

R can be derived from suitable starting fatty acids such as lauric acid (C12), myristic acid (C14), palmitic acid (C16), stearic acid (C18), oleic acid (C18:1), and mixtures thereof. If the starting fatty acid is naturally derived, the starting fatty acid will typically have a distribution of chain lengths, such as from 8 to 18 carbon atoms, or narrower if refined to achieve the desired alkyl chain length distributions. Lauric acid (C12), myristic acid (C14), and especially mixtures thereof are preferred. Since the carbon chain count of the fatty acid includes the carbon atom of the carboxylic acid of the fatty acid, the carbon count of R in formula (I) of the alkyl alkanolamide sulfate surfactant is one less than the carbon count of the fatty acid. For instance, if the alkyl alkanolamide surfactant was formed using lauric acid (C12), R in R in formula (I) of the alkyl alkanolamide sulfate surfactant would be C11 alkyl.

The alkyl chain R can have a mol percentage of C11 to C13 chains to total alkyl chains of at least 60%, preferably at least 70%, more preferably at least 80%, most preferably at least 90%. The molar ratio of C11 to C13 alkyl chains in R can be from 1:2 to 6:1, preferably from 1: 1 to 5: 1.

R can be a linear alkyl chain. For instance, to improve grease cleaning. As such, R can be naturally derived from renewable feedstock such as coconut oil, palm kernel oil, and mixtures thereof, with coconut oil being preferred.

Alternatively, but less preferred alkyl chain of R can be branched. For instance, to improve low temperature stability of the resultant detergent composition. A combination of good grease cleaning and improved low temperature stability of the detergent composition can be achieved when alkyl chains having a weight average degree of branching of at least 15 %, preferably from 20 % to 60 %, more preferably from 30 % to 50 %, is used for R. The weight average degree of branching for the R group is calculated using the same methodology as described later for alkyl sulfate anionic surfactants. The weight average degree of branching and the distribution of branching can typically be obtained from the technical data sheet for the constituent fatty acid used to make the alkyl alkanolamide sulfate anionic surfactant. Alternatively, the type of branching can also be determined through analytical methods known in the art, including capillary gas chromatography with flame ionisation detection on medium polar capillary column, using hexane as the solvent.

The alkyl alkanolamide sulfate anionic surfactant preferably comprises C12 alkyl alkanolamide sulfate anionic surfactant, C14 alkyl alkanolamide sulfate anionic surfactant, and mixtures thereof, with a blend of C12 alkyl alkanolamide sulfate anionic surfactant and C14 alkyl alkanolamide sulfate anionic surfactant being preferred. As such, R in formula (I) is preferably C11 or C13, or a combination thereof.

Suitable C12 alkyl alkanolamide sulfate anionic surfactant can be selected from the group consisting of: N-(2-hydroxyethyl)dodecanamide sulfate, N-(2-hydroxypropyl)dodecanamide sulfate, N-(2-hydroxyethyl)-N-methyldodecanamide sulfate, N-(1-hydroxypropan-2-yl)dodecanamide sulfate, and mixtures thereof, preferably N-(2-hydroxyethyl) dodecanamide sulfate, N-(2-hydroxypropyl)dodecanamide sulfate, N-(2-hydroxyethyl)-N-methyldodecanamide sulfate, and mixtures thereof.

Suitable C14 alkyl alkanolamide sulfate anionic surfactant can be selected from the group consisting of: N-(2-hydroxyethyl)tetradecanamide sulfate, N-(2-hydroxypropyl)tetradecanamide sulfate, N-(2-hydroxyethyl)-N-methyltetradecanamide sulfate, N-(1-hydroxypropan-2-yl) tetradecanamide sulfate, and mixtures thereof, preferably N-(2-hydroxyethyl) tetradecanamide sulfate, N-(2-hydroxypropyl) tetradecanamide sulfate, N-(2-hydroxyethyl)-N-methyltetradecanamide sulfate, and mixtures thereof.

Alkyl sulfate anionic surfactant

The anionic surfactant preferably further comprises alkyl sulfate anionic surfactant. The anionic surfactant can comprise at least 25%, preferably from 30% to 90%, more preferably from 65% to 85% by weight of the anionic surfactant of alkyl sulfate anionic surfactant.

The anionic surfactant can comprise at least 70%, more preferably at least 85%, most preferably 100% by weight of the anionic surfactant of the alkyl sulfate anionic surfactant and the alkyl alkanolamide sulfate anionic surfactant. In preferred compositions, the anionic surfactant consists of alkyl sulfate anionic surfactant and the alkanolamide sulfate anionic surfactant. The alkyl sulfate anionic surfactant and the alkyl alkanolamide sulfate anionic surfactant are preferably present at a weight ratio of from 10:1 to 1:2, preferably from 7:1 to 1:1, and most preferably from 5:1 to 2:1.

Without wishing to be bound by theory, it is believed that a mixture provides a surfactant packing which balances grease cleaning and suds mileage performance, especially in presence of greasy-particulate soils, low temperature stability and demonstrating a minimum impact on the targeted finished product viscosity.

The mol average alkyl chain length of the alkyl sulfate anionic surfactant can be from 8 to 18, preferably from 10 to

14, more preferably from 12 to 14, most preferably from 12 to 13 carbon atoms, in order to provide a combination of improved grease removal and enhanced speed of cleaning.

The alkyl chain of the alkyl sulfate anionic surfactant can have a mol fraction of C12 and C13 chains of at least 50%, preferably at least 65%, more preferably at least 80%, most preferably at least 90%. Suds mileage is particularly improved, especially in the presence of greasy-particulate soils, when the C13/C12 mol ratio of the alkyl chain is at least 57/43, preferably from 60/40 to 90/10, more preferably from 60/40 to 80/20, most preferably from 60/40 to 70/30, while not compromising suds mileage in the presence of particulate soils.

The relative molar amounts of C13 and C12 alkyl chains in the alkyl sulfate anionic surfactant can be derived from the carbon chain length distribution of the anionic surfactant. The carbon chain length distribution of the alkyl chains of the alkyl sulfate anionic surfactants can be obtained from the technical data sheets from the suppliers for the surfactant or constituent alkyl alcohol. Alternatively, the chain length distribution and average molecular weight of the fatty alcohols, used to make the alkyl sulfate anionic surfactant, can also be determined by methods known in the art. Such methods include capillary gas chromatography with flame ionisation detection on medium polar capillary column, using hexane as the solvent. The chain length distribution is based on the starting alcohol and alkoxylated alcohol. As such, the alkyl sulfate anionic surfactant should be hydrolysed back to the corresponding alkyl alcohol and alkyl alkoxylated alcohol before analysis, for instance using hydrochloric acid.

The alkyl sulfate anionic surfactant can have a weight average degree of branching of at least 15%, preferably from 20% to 60%, more preferably from 30% to 50%. Compositions comprising such branched alkyl sulfate anionic surfactants typically have improved viscosity control and low temperature stability. More preferably, the alkyl sulfate anionic surfactant has an average degree of branching of less than 15%, more preferably less than 10%, most preferably the alkyl sulfate anionic surfactant is linear. Linear alkyl chains are typically derived from renewable sources.

The alkyl sulfate anionic surfactant can comprise at least 5%, preferably at least 10%, most preferably at least 25%, by weight of the alkyl sulfate anionic surfactant, of branching on the C2 position (as measured counting carbon atoms from the sulfate group for non-alkoxylated alkyl sulfate anionic surfactants, and the counting from the alkoxy-group furthest from the sulfate group for alkoxylated alkyl sulfate anionic surfactants). More preferably, greater than 75%, even more preferably greater than 90%, by weight of the total branched alkyl content consists of C1-C5 alkyl moiety, preferably C1-C2 alkyl moiety. It has been found that formulating the inventive compositions using alkyl sulfate surfactants having the aforementioned degree of branching results in improved low temperature stability. Such compositions require less solvent in order to achieve good physical stability at low temperatures. As such, the compositions can comprise lower levels of organic solvent, of less than 5.0% by weight of the liquid cleaning composition of organic solvent, while still having improved low temperature stability. Higher surfactant branching also provides faster initial suds generation, but typically less suds mileage. The weight average branching, described herein, has been found to provide improved low temperature stability, initial foam generation and suds longevity.

The weight average degree of branching for an anionic surfactant mixture can be calculated using the following formula:

Weight average degree of branching (%) = [(x1 * wt% branched alcohol 1 in alcohol 1 + x2 * wt% branched alcohol 2 in alcohol 2 + ....) / (x1 + x2 + ....)] * 100

wherein x1, x2, ... are the weight in grams of each alcohol in the total alcohol mixture of the alcohols which were used as starting material before (alkoxylation and) sulfation to produce the alkyl (alkoxy) sulfate anionic surfactant. In the weight average degree of branching calculation, the weight of the alkyl alcohol used to form the alkyl sulfate anionic surfactant which is not branched is included.

The weight average degree of branching and the distribution of branching can typically be obtained from the technical data sheet for the surfactant or constituent alkyl alcohol. Alternatively, the branching can also be determined through analytical methods known in the art, including capillary gas chromatography with flame ionisation detection on medium polar capillary column, using hexane as the solvent. The weight average degree of branching and the distribution of branching is based on the starting alcohol used to produce the alkyl sulfate anionic surfactant.

The alkyl sulfate surfactant can be alkoxylated or free of alkoxylation.

When alkoxylated, the alkyl sulfate anionic surfactant can have an average degree of alkoxylation of less than 0.1, with no alkoxylation being particularly preferred. When alkoxylated, ethoxylation is preferred.

As such, the alkyl sulfate anionic surfactant comprises less than 10% preferably less than 5% by weight of the alkyl sulfate anionic surfactant of an alkoxylated alkyl sulfate surfactant, more preferably wherein the alkyl sulfate anionic surfactant is free of an alkoxylated alkyl sulfate surfactant.

The average degree of alkoxylation is the mol average degree of alkoxylation (i.e., mol average alkoxylation degree) of all the alkyl sulfate anionic surfactant. Hence, when calculating the mol average alkoxylation degree, the mols of non-alkoxylated sulfate anionic surfactant are included:

Mol average alkoxylation degree = (x1 * alkoxylation degree of surfactant 1 + x2 * alkoxylation degree of surfactant 2 + ....) / (x1 + x2 + ....)

wherein x1, x2, ... are the number of moles of each alkyl (or alkoxy) sulfate anionic surfactant of the mixture and alkoxylation degree is the number of alkoxy groups in each alkyl sulfate anionic surfactant.

If used, preferred alkyl alkoxy sulfates are alkyl ethoxy sulfates.

Non-alkoxylated alkyl sulfate surfactants can be formed using naturally derived alkyl chains, such as those derived from palm oil or coconut oil. It has also been found that non-alkoxylated alkyl sulfate surfactants are more readily biodegradable by microorganisms in soil and natural waters. However, such naturally derived alkyl chains are typically fully linear, resulting in fully linear non-alkoxylated alkyl sulfate surfactants. Liquid detergent compositions comprising linear alkyl sulfates typically require more solvent to provide the desired low temperature phase stability and to achieve the desired viscosity profile for ease of dosing by the user. The increased solvent also results in a less environmentally sustainable composition.

Suitable counterions include alkali metal cation, earth alkali metal cation, alkanolammonium or ammonium or substituted ammonium, but preferably sodium, since the use of alkanolammonium or ammonium or substituted ammonium can lead to discoloration of the composition.

Suitable examples of commercially available alkyl sulfate anionic surfactants include, those derived from alcohols sold under the Neodol® brand-name by Shell, or the Lial®, Isalchem®, and Safol® brand-names by Sasol, or some of the natural alcohols produced by The Procter & Gamble Chemicals company. The alcohols can be blended in order to achieve the desired mol fraction of C12 and C13 chains and the desired C13/C12 ratio, based on the relative fractions of C13 and C12 within the starting alcohols, as obtained from the technical data sheets from the suppliers or from analysis using methods known in the art.

The performance can be affected by the width of the alkoxylation distribution of the alkoxylated alkyl sulfate anionic surfactant, including grease cleaning, sudsing, low temperature stability and viscosity of the finished product. The alkoxylation distribution, including its broadness can be varied through the selection of catalyst and process conditions when making the alkoxylated alkyl sulfate anionic surfactant.

If ethoxylated alkyl sulfate is present, without wishing to be bound by theory, through tight control of processing conditions and feedstock material compositions, both during alkoxylation especially ethoxylation and sulfation steps, the amount of 1,4-dioxane by-product within alkoxylated especially ethoxylated alkyl sulfates can be reduced. Based on recent advances in technology, a further reduction of 1,4-dioxane by-product can be achieved by subsequent stripping, distillation, evaporation, centrifugation, microwave irradiation, molecular sieving or catalytic or enzymatic degradation steps. Processes to control 1,4-dioxane content within alkoxylated/ethoxylated alkyl sulfates have been described extensively in the art. Alternatively 1,4-dioxane level control within detergent formulations has also been described in the art through addition of 1,4-dioxane inhibitors to 1,4-dioxane comprising formulations, such as 5,6-dihydro-3-(4-morpholinyl)-1-[4-(2-oxo-1-piperidinyl)-phenyl]-2-(1-H)-pyridone, 3-$\alpha$-hydroxy-7-oxo stereoisomer-mixtures of cholinic acid, 3-(N- methyl amino)-L-alanine, and mixtures thereof.

Additional anionic surfactant

The anionic surfactant can comprise additional anionic surfactant such as those selected from the group consisting of: alkyl (benzene) sulfonate surfactant, alkyl sulfosuccinate and dialkyl sulfosuccinate ester surfactants, and mixtures thereof.

Anionic alkyl sulfonate or sulfonic acid surfactants suitable for use herein include the acid and salt forms of alkylbenzene sulfonates, alkyl ester sulfonates, primary and secondary alkane sulfonates such as paraffin sulfonates, alfa or internal olefin sulfonates, alkyl sulfonated (poly)carboxylic acids, and mixtures thereof. Suitable anionic sulfonate or sulfonic acid surfactants include: C5-C20 alkylbenzene sulfonates, more preferably C10-C16 alkylbenzene sulfonates, more preferably C11-C13 alkylbenzene sulfonates, C5-C20 alkyl ester sulfonates especially C5-C20 methyl ester sulfonates, C6-C22 primary or secondary alkane sulfonates, C5-C20 sulfonated (poly)carboxylic acids, and any mixtures thereof, but preferably C11-C13 alkylbenzene sulfonates. The aforementioned surfactants can vary widely in their 2-phenyl isomer content. Compared with sulfonation of alpha olefins, the sulfonation of internal olefins can occur at any position since the double bond is randomly positioned, which leads to the position of hydrophilic sulfonate and hydroxyl groups of IOS in the middle of the alkyl chain, resulting in a variety of twin-tailed branching structures. Alkane sulfonates include paraffin sulfonates and other secondary alkane sulfonate (such as Hostapur SAS60 from Clariant).

Alkyl sulfosuccinate and dialkyl sulfosuccinate esters are organic compounds with the formula MO3SCH(CO2R')CH2CO2R where R and R' can be H or alkyl groups, and M is a counter-ion such as sodium (Na). Alkyl sulfosuccinate and dialkyl sulfosuccinate ester surfactants can be alkoxylated or non-alkoxylated, preferably non-alkoxylated. The surfactant system may comprise further anionic surfactant. However, the composition preferably comprises less than 30%, preferably less than 15%, more preferably less than 10% by weight of the surfactant system

of further anionic surfactant. Most preferably, the surfactant system comprises no further anionic surfactant, preferably no other anionic surfactant than alkyl sulfate anionic surfactant and the acyl taurate anionic surfactant.

Co-Surfactant

In order to improve surfactant packing after dilution and hence improve suds mileage, the surfactant system can comprise a co-surfactant which is selected from the group consisting of an amphoteric surfactant, a zwitterionic surfactant and mixtures thereof.

The anionic surfactant to the co-surfactant weight ratio can be from 1:1 to 8:1, preferably from 2:1 to 5:1, more preferably from 2.5:1 to 4:1.

The composition preferably comprises from 0.1% to 20%, more preferably from 0.5% to 15% and especially from 2% to 10% by weight of the cleaning composition of the co-surfactant.

The surfactant system of the cleaning composition of the present invention preferably comprises up to 50%, preferably from 10% to 40%, more preferably from 15% to 35%, by weight of the surfactant system of a co-surfactant.

The co-surfactant is preferably an amphoteric surfactant, more preferably an amine oxide surfactant.

The amine oxide surfactant can be linear or branched, though linear are preferred. Suitable linear amine oxides are typically water-soluble, and characterized by the formula R1 - N(R2)(R3) O. R1 is a C8-18 alkyl, R1 is preferably is a linear alkyl chain, more preferably derived from natural, renewable resources such as coconut or palm kernel, with coconut being particularly preferred. R2 and R3 moieties are selected from the group consisting of C1-3 alkyl groups, C1-3 hydroxyalkyl groups, and mixtures thereof. For instance, R2 and R3 can be selected from the group consisting of: methyl, ethyl, propyl, isopropyl, 2-hydroxethyl, 2-hydroxypropyl and 3-hydroxypropyl, and mixtures thereof, though methyl is preferred for one or both of R2 and R3. The linear amine oxide surfactants in particular may include linear C10-C18 alkyl dimethyl amine oxides and linear C8-C12 alkoxy ethyl dihydroxy ethyl amine oxides.

Preferably, the amine oxide surfactant is selected from the group consisting of: alkyl dimethyl amine oxide, alkyl amido propyl dimethyl amine oxide, and mixtures thereof. Alkyl dimethyl amine oxides are particularly preferred, such as C8-18 alkyl dimethyl amine oxides, or C10-16 alkyl dimethyl amine oxides (such as coco dimethyl amine oxide). Suitable alkyl dimethyl amine oxides include C10 alkyl dimethyl amine oxide surfactant, C10-12 alkyl dimethyl amine oxide surfactant, C12-C14 alkyl dimethyl amine oxide surfactant, and mixtures thereof. C12-C14 alkyl dimethyl amine oxide are particularly preferred.

Alternative suitable amine oxide surfactants include mid-branched amine oxide surfactants. As used herein, "mid-branched" means that the amine oxide has one alkyl moiety having n1 carbon atoms with one alkyl branch on the alkyl moiety having n2 carbon atoms. The alkyl branch is located on the $\alpha$ carbon from the nitrogen on the alkyl moiety. This type of branching for the amine oxide is also known in the art as an internal amine oxide. The total sum of n1 and n2 can be from 10 to 24 carbon atoms, preferably from 12 to 20, and more preferably from 10 to 16. The number of carbon atoms for the one alkyl moiety (n1) is preferably the same or similar to the number of carbon atoms as the one alkyl branch (n2) such that the one alkyl moiety and the one alkyl branch are symmetric. As used herein "symmetric" means that | n1 - n2 | is less than or equal to 5, preferably 4, most preferably from 0 to 4 carbon atoms in at least 50 wt%, more preferably at least 75 wt% to 100 wt% of the mid-branched amine oxides for use herein. The amine oxide further comprises two moieties, independently selected from a C1-3 alkyl, a C1-3 hydroxyalkyl group, or a polyethylene oxide group containing an average of from about 1 to about 3 ethylene oxide groups. Preferably, the two moieties are selected from a C1-3 alkyl, more preferably both are selected as C1 alkyl.

Alternatively, the amine oxide surfactant can be a mixture of amine oxides comprising a mixture of low-cut amine oxide and mid-cut amine oxide. The amine oxide of the composition of the invention can then comprises:

a) from about 10% to about 45% by weight of the amine oxide of low-cut amine oxide of formula R1R2R3AO wherein R1 and R2 are independently selected from hydrogen, C1-C4 alkyls or mixtures thereof, and R3 is selected from C10 alkyls and mixtures thereof; and

b) from 55% to 90% by weight of the amine oxide of mid-cut amine oxide of formula R4R5R6AO wherein R4 and R5 are independently selected from hydrogen, C1-C4 alkyls or mixtures thereof, and R6 is selected from C12-C16 alkyls or mixtures thereof

In a preferred low-cut amine oxide for use herein R3 is n-decyl, with preferably both R1 and R2 being methyl. In the mid-cut amine oxide of formula R4R5R6AO, R4 and R5 are preferably both methyl.

Preferably, the amine oxide comprises less than about 5%, more preferably less than 3%, by weight of the amine oxide of an amine oxide of formula R7R8R9AO wherein R7 and R8 are selected from hydrogen, C1-C4 alkyls and mixtures thereof and wherein R9 is selected from C8 alkyls and mixtures thereof. Limiting the amount of amine oxides of formula R7R8R9AO improves both physical stability and suds mileage.

Suitable zwitterionic surfactants include betaine surfactants. Such betaine surfactants includes alkyl betaines,

alkylamidobetaines, amidazoliniumbetaines, sulfobetaine (INCI Sultaines), phosphobetaines, and mixtures thereof, and preferably meets formula (I):

$$R^1\text{-}[CO\text{-}X(CH_2)_n]_x\text{-}N^+(R^2)(R_3)\text{-}(CH_2)_m\text{-}[CH(OH)\text{-}CH_2]_y\text{-}Y^-$$

Wherein in formula (I),

R1 is selected from the group consisting of: a saturated or unsaturated C6-22 alkyl residue, preferably C8-18 alkyl residue, more preferably a saturated C10-16 alkyl residue, most preferably a saturated C12-14 alkyl residue; R1 is preferably a linear alkyl chain, preferably derived from natural, renewable resources such as coconut or palm kernel, preferably coconut.
X is selected from the group consisting of: NH, NR4 wherein R4 is a C1-4 alkyl residue, O, and S,
n is an integer from 1 to 10, preferably 2 to 5, more preferably 3,
x is 0 or 1, preferably 1,
R2 and R3 are independently selected from the group consisting of: a C1-4 alkyl residue, hydroxy substituted such as a hydroxyethyl, and mixtures thereof, preferably both R2 and R3 are methyl,
m is an integer from 1 to 4, preferably 1, 2 or 3,
y is 0 or 1, and
Y is selected from the group consisting of: COO, SO3, OPO(ORS)O or P(O)(OR5)O, wherein R5 is H or a C1-4 alkyl residue.

Preferred betaines are the alkyl betaines of formula (Ia), the alkyl amido propyl betaine of formula (Ib), the sulfobetaine of formula (Ic) and the amido sulfobetaine of formula (Id):

$$R^1\text{-}N^+(CH_3)_2\text{-}CH_2COO^- \quad (IIa)$$

$$R^1\text{-}CO\text{-}NH\text{-}(CH_2)_3\text{-}N^+(CH_3)_2\text{-}CH_2COO^- \quad (IIb)$$

$$R^1N^+(CH_3)_2\text{-}CH_2CH(OH)CH_2SO_3^- \quad (IIc)$$

$$R^1\text{-}CO\text{-}NH\text{-}(CH_2)_3\text{-}N^+(CH_3)_2\text{-}CH_2CH(OH)CH_2SO_3^- \quad (IId)$$

in which R1 has the same meaning as in formula (I). Particularly preferred are the carbobetaines [i.e. wherein Y-=COO- in formula (I)] of formulae (Ia) and (Ib), more preferred are the alkylamidobetaine of formula (Ib).
Suitable betaines can be selected from the group consisting or [designated in accordance with INCI]: capryl/-capramidopropyl betaine, cetyl betaine, cetyl amidopropyl betaine, cocamidoethyl betaine, cocamidopropyl betaine, cocobetaines, decyl betaine, decyl amidopropyl betaine, hydrogenated tallow betaine / amidopropyl betaine, isostearamidopropyl betaine, lauramidopropyl betaine, lauryl betaine, myristyl amidopropyl betaine, myristyl betaine, oleamidopropyl betaine, oleyl betaine, palmamidopropyl betaine, palmitamidopropyl betaine, palm-kernelamidopropyl betaine, stearamidopropyl betaine, stearyl betaine, tallowamidopropyl betaine, tallow betaine, undecylenamidopropyl betaine, undecyl betaine, and mixtures thereof. Preferred betaines are selected from the group consisting of: cocamidopropyl betaine, cocobetaines, lauramidopropyl betaine, lauryl betaine, myristyl amidopropyl betaine, myristyl betaine, and mixtures thereof. Cocamidopropyl betaine and/or laurylamidopropylbetaine are particularly preferred.

Nonionic Surfactant

The surfactant system can further comprise a nonionic surfactant. Suitable nonionic surfactants include alkoxylated alcohol nonionic surfactants, alkyl polyglucoside nonionic surfactants, and mixtures thereof. Where the nonionic surfactant comprises a blend of alkyl polyglucoside and alkoxylated alcohol nonionic surfactant, the nonionic surfactant can comprise the alkyl polyglucoside and alkoxylated alcohol nonionic surfactant in a mass ratio of from 10:90 to 90: 10, preferably from 30:70 to 70:30, more preferably from 40:60 to 60:40.
The surfactant system of the composition of the present invention can further comprise from 1.0% to 50%, preferably from 1.25% to 25%, more preferably from 1.5% to 15%, most preferably from 1.5% to 5%, by weight of the surfactant system, of nonionic surfactant.

Alkoxylated alcohol nonionic surfactant:

Preferably, the alkoxylated alcohol non-ionic surfactant is a linear or branched, primary or secondary alkyl

alkoxylated non-ionic surfactant, preferably an alkyl ethoxylated non-ionic surfactant, preferably comprising on average from 9 to 15, preferably from 10 to 14 carbon atoms in its alkyl chain and on average from 5 to 12, preferably from 6 to 10, most preferably from 7 to 8, units of ethylene oxide per mole of alcohol. The alkyl chain is preferably linear.

Suitable examples of commercially available alkoxylated alcohol nonionic surfactants include, those derived from alcohols sold under the Neodol® brand-name by Shell, or the Lial®, Isalchem®, and Safol® brand-names by Sasol, or some of the natural alcohols produced by The Procter & Gamble Chemicals company. The performance can be affected by the width of the alkoxylation distribution of the alkoxylated alcohol nonionic surfactant. The alkoxylation distribution, including its broadness can be varied through the selection of catalyst and process conditions when making the alkoxylated alcohol nonionic surfactant.

Alkyl polyglucoside nonionic surfactant:

Alkyl polyglucoside nonionic surfactants are typically more sudsing than other nonionic surfactants such as alkyl ethoxylated alcohols.

A combination of alkylpolyglucoside and anionic surfactant especially a mixture of alkyl sulfate and alkyl alkanolamide sulfate anionic surfactant, has been found to improve polymerized grease removal, suds mileage performance, reduced viscosity variation with changes in the surfactant and/or system, and a more sustained Newtonian rheology.

The alkyl polyglucoside surfactant can be selected from C6-C18 alkyl polyglucoside surfactant. The alkyl polyglucoside surfactant can have a number average degree of polymerization of from 0.1 to 3.0, preferably from 1.0 to 2.0, more preferably from 1.2 to 1.6. The alkyl polyglucoside surfactant can comprise a blend of short chain alkyl polyglucoside surfactant having an alkyl chain comprising 10 carbon atoms or less, and mid to long chain alkyl polyglucoside surfactant having an alkyl chain comprising greater than 10 carbon atoms to 18 carbon atoms, preferably from 12 to 14 carbon atoms. The alkyl chain is preferably linear.

Short chain alkyl polyglucoside surfactants have a monomodal chain length distribution between C8-C10, mid to long chain alkyl polyglucoside surfactants have a monomodal chain length distribution between C10-C18, while mid chain alkyl polyglucoside surfactants have a monomodal chain length distribution between C12-C14. In contrast, C8 to C18 alkyl polyglucoside surfactants typically have a monomodal distribution of alkyl chains between C8 and C18, as with C8 to C16 and the like. As such, a combination of short chain alkyl polyglucoside surfactants with mid to long chain or mid chain alkyl polyglucoside surfactants have a broader distribution of chain lengths, or even a bimodal distribution, than non-blended C8 to C18 alkyl polyglucoside surfactants. Preferably, the weight ratio of short chain alkyl polyglucoside surfactant to long chain alkyl polyglucoside surfactant is from 1:1 to 10:1, preferably from 1.5:1 to 5:1, more preferably from 2:1 to 4:1. It has been found that a blend of such short chain alkyl polyglucoside surfactant and long chain alkyl polyglucoside surfactant results in faster dissolution of the detergent solution in water and improved initial sudsing, in combination with improved suds stability.

C8-C16 alkyl polyglucosides are commercially available from several suppliers (e.g., Simusol® surfactants from Seppic Corporation; and Glucopon® 600 CSUP, Glucopon® 650 EC, Glucopon® 600 CSUP/MB, and Glucopon® 650 EC/MB, from BASF Corporation). Glucopon® 215UP is a preferred short chain APG surfactant. Glucopon® 600CSUP is a preferred mid to long chain APG surfactant.

In preferred compositions, the surfactant system can comprise an alkyl sulfate anionic surfactant and an acyl taurate anionic surfactant having an average degree of branching of less than 10%, and alkyl polyglucoside nonionic surfactant.

Further ingredients

The cleaning composition may optionally comprise a number of other adjunct ingredients such as builders (preferably citrate), chelants, conditioning polymers, other cleaning polymers, surface modifying polymers, structurants, emollients, humectants, skin rejuvenating actives, enzymes, carboxylic acids, scrubbing particles, perfumes, malodor control agents, pigments, dyes, opacifiers, pearlescent particles, inorganic cations such as alkaline earth metals such as Ca/Mg-ions, antibacterial agents, preservatives, viscosity adjusters (*e.g.*, salt such as NaCl, and other mono-, di- and trivalent salts) and pH adjusters and buffering means (*e.g.* carboxylic acids such as citric acid, HCl, NaOH, KOH, alkanolamines, carbonates such as sodium carbonates, bicarbonates, sesquicarbonates, and alike).

Preferred further ingredients include those selected from: amphiphilic alkoxylated polyalkyleneimines, cyclic polyamines, triblock copolymers, hydroxypropylcellulose polymers, salt, hydrotropes, organic solvents, and mixtures thereof.

Amphiphilic alkoxylated polyalkyleneimine:

The composition of the present invention may further comprise from 0.05% to 2%, preferably from 0.07% to 1% by weight of the total composition of an amphiphilic polymer. Suitable amphiphilic polymers can be selected from the group consisting of: amphiphilic alkoxylated polyalkyleneimine and mixtures thereof. The amphiphilic alkoxylated polyalkyleneimine polymer has been found to reduce gel formation on the hard surfaces to be cleaned when the liquid composition is added directly to a cleaning implement (such as a sponge) before cleaning and consequently brought in contact with heavily greased surfaces, especially when the cleaning implement comprises a low amount to nil water such as when light pre-wetted sponges are used.

A preferred amphiphilic alkoxylated polyethyleneimine polymer has the general structure of formula (I):

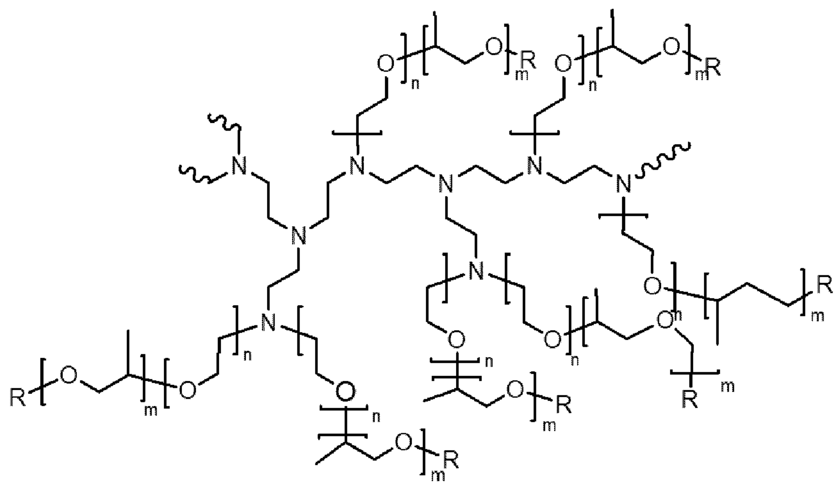

(I)

wherein the polyethyleneimine backbone has a weight average molecular weight of 600, n of formula (I) has an average of 10, m of formula (I) has an average of 7 and R of formula (I) is selected from hydrogen, a $C_1$-$C_4$ alkyl and mixtures thereof, preferably hydrogen. The degree of permanent quaternization of formula (I) may be from 0% to 22% of the polyethyleneimine backbone nitrogen atoms. The molecular weight of this amphiphilic alkoxylated polyethyleneimine polymer preferably is between 10,000 and 15,000 Da.

More preferably, the amphiphilic alkoxylated polyethyleneimine polymer has the general structure of formula (I) but wherein the polyethyleneimine backbone has a weight average molecular weight of 600 Da, n of Formula (I) has an average of 24, m of Formula (I) has an average of 16 and R of Formula (I) is selected from hydrogen, a $C_1$-$C_4$ alkyl and mixtures thereof, preferably hydrogen. The degree of permanent quaternization of Formula (I) may be from 0% to 22% of the polyethyleneimine backbone nitrogen atoms and is preferably 0%. The molecular weight of this amphiphilic alkoxylated polyethyleneimine polymer preferably is between 25,000 and 30,000, most preferably 28,000 Da.

The amphiphilic alkoxylated polyethyleneimine polymers can be made by the methods described in more detail in PCT Publication No. WO 2007/135645.

Alternatively, the compositions can be free of amphiphilic polymers.

Cyclic Polyamine

The composition can comprise a cyclic polyamine having amine functionalities that helps cleaning. The composition of the invention preferably comprises from 0.1% to 3%, more preferably from 0.2% to 2%, and especially from 0.5% to 1%, by weight of the total composition, of the cyclic polyamine.

The cyclic polyamine has at least two primary amine functionalities. The primary amines can be in any position in the cyclic amine but it has been found that in terms of grease cleaning, better performance is obtained when the primary amines are in positions 1,3. It has also been found that cyclic amines in which one of the substituents is -CH3 and the rest are H provided for improved grease cleaning performance.

Accordingly, the most preferred cyclic polyamine for use with the cleaning composition of the present invention are cyclic polyamine selected from the group consisting of: 2-methylcyclohexane-1,3-diamine, 4-methylcyclohexane-1,3-diamine and mixtures thereof. These specific cyclic polyamines work to improve suds and grease cleaning profile throughout the dishwashing process when formulated together with the surfactant system of the composition of the present invention.

Suitable cyclic polyamines can be supplied by BASF, under the Baxxodur tradename, with Baxxodur ECX-210

being particularly preferred.

A combination of the cyclic polyamine and magnesium sulfate is particularly preferred. As such, the composition can further comprise magnesium sulfate at a level of from 0.001 % to 2.0 %, preferably from 0.005 % to 1.0 %, more preferably from 0.01 % to 0.5 % by weight of the composition.

Triblock Copolymer

The composition of the invention can comprise a triblock copolymer. The triblock co-polymers can be present at a level of from 1% to 20%, preferably from 3% to 15%, more preferably from 5% to 12%, by weight of the total composition. Suitable triblock copolymers include alkylene oxide triblock co-polymers, defined as a triblock co-polymer having alkylene oxide moieties according to Formula (I): (EO)x(PO)y(EO)x, wherein EO represents ethylene oxide, and each x represents the number of EO units within the EO block. Each x can independently be on average of from 5 to 50, preferably from 10 to 40, more preferably from 10 to 30. Preferably x is the same for both EO blocks, wherein the "same" means that the x between the two EO blocks varies within a maximum 2 units, preferably within a maximum of 1 unit, more preferably both x's are the same number of units. PO represents propylene oxide, and y represents the number of PO units in the PO block. Each y can on average be from between 28 to 60, preferably from 30 to 55, more preferably from 30 to 48.

Preferably the triblock co-polymer has a ratio of y to each x of from 3:1 to 2:1. The triblock co-polymer preferably has a ratio of y to the average x of 2 EO blocks of from 3:1 to 2:1. Preferably the triblock co-polymer has an average weight percentage of total E-O of between 30% and 50% by weight of the tri-block co-polymer. Preferably the triblock co-polymer has an average weight percentage of total PO of between 50% and 70% by weight of the triblock co-polymer. It is understood that the average total weight % of EO and PO for the triblock co-polymer adds up to 100%. The triblock co-polymer can have an average molecular weight of between 2060 and 7880, preferably between 2620 and 6710, more preferably between 2620 and 5430, most preferably between 2800 and 4700. Average molecular weight is determined using a 1H NMR spectroscopy (*see* Thermo scientific application note No. AN52907).

Triblock co-polymers have the basic structure ABA, wherein A and B are different homopolymeric and/or monomeric units. In this case A is ethylene oxide (EO) and B is propylene oxide (PO). Those skilled in the art will recognize the phrase "block copolymers" is synonymous with this definition of "block polymers".

Triblock co-polymers according to Formula (I) with the specific EO/PO/EO arrangement and respective homopolymeric lengths have been found to enhances suds mileage performance of the liquid hand dishwashing detergent composition in the presence of greasy soils and/or suds consistency throughout dilution in the wash process.

Suitable EO-PO-EO triblock co-polymers are commercially available from BASF such as Pluronic® PE series, and from the Dow Chemical Company such as Tergitol™ L series. Particularly preferred triblock co-polymer from BASF are sold under the tradenames Pluronic® PE6400 (MW ca 2900, ca 40wt% EO) and Pluronic® PE 9400 (MW ca 4600, 40 wt% EO). Particularly preferred triblock co-polymer from the Dow Chemical Company is sold under the tradename Tergitol™ L64 (MW ca 2700, ca 40 wt% EO).

Preferred triblock co-polymers are readily biodegradable under aerobic conditions.

Salt:

The composition of the present invention may comprise from about 0.05% to about 2%, preferably from about 0.1% to about 1.5%, or more preferably from about 0.5% to about 1%, by weight of the total composition of a salt, preferably a monovalent or divalent inorganic salt, or a mixture thereof, more preferably selected from: sodium chloride, sodium sulfate, and mixtures thereof. Sodium chloride is most preferred.

Hydrotrope:

The composition of the present invention may comprise from about 0.1% to about 10%, or preferably from about 0.5% to about 10%, or more preferably from about 1% to about 10% by weight of the total composition of a hydrotrope or a mixture thereof, preferably sodium cumene sulfonate.

Organic Solvent:

The composition can comprise from about 0.1% to about 10%, or preferably from about 0.5% to about 10%, or more preferably from about 1% to about 10% by weight of the total composition of an organic solvent. Suitable organic solvents include organic solvents selected from the group consisting of: alcohols, glycols, glycol ethers, and mixtures thereof, preferably alcohols, glycols, and mixtures thereof. Ethanol is the preferred alcohol. Polyalkyleneglycols, especially polypropyleneglycol, is the preferred glycol, with polypropyleneglycols having a weight average molecular weight of from 750 Da to 1,400 Da being particularly preferred.

Packaged product

The hand dishwashing detergent composition can be packaged in a container, typically plastic containers. Suitable containers comprise an orifice. Typically, the container comprises a cap, with the orifice typically comprised on the cap. The cap can comprise a spout, with the orifice at the exit of the spout. The spout can have a length of from 0.5 mm to 10 mm.

The orifice can have an open cross-sectional surface area at the exit of from 3 $mm^2$ to 20 $mm^2$, preferably from 3.8 $mm^2$ to 12 $mm^2$, more preferably from 5 $mm^2$ to 10 $mm^2$, wherein the container further comprises the composition according to the invention. The cross-sectional surface area is measured perpendicular to the liquid exit from the container (that is, perpendicular to the liquid flow during dispensing).

The container can typically comprise from 200 ml to 5,000 ml, preferably from 350 ml to 2000 ml, more preferably from 400 ml to 1,000 ml of the liquid hand dishwashing detergent composition.

Alternatively, the hand dishwashing detergent composition can be packaged in an inverted container. Such inverted containers typically comprise a cap at the bottom of the container, the cap comprising either a closure or a self-sealing valve, or a combination thereof. The cap preferably comprises a self-sealing valve. Suitable self-sealing valves include slit-valves. The self-sealing valve defines a dispensing orifice that is reactively openable when the pressure on the valve interior side exceeds the pressure on the valve exterior side. The bottom dispensing container can comprise an impact resistance system, such as that described in WO2019108293A1.

Method of Washing

The invention is further directed to a method of manually washing dishware with the composition of the present invention. The method comprises the steps of delivering a composition of the present invention to a volume of water to form a wash solution and immersing the dishware in the solution. The dishware is be cleaned with the composition in the presence of water.

Optionally, the dishware can be rinsed. By "rinsing", it is meant herein contacting the dishware cleaned with the process according to the present invention with substantial quantities of appropriate solvent, typically water. By "substantial quantities", it is meant usually about 1 to about 20 L, or under running water.

The composition herein can be applied in its diluted form. Soiled dishware is contacted with an effective amount, typically from about 0.5 mL to about 20 mL (per about 25 dishes being treated), preferably from about 3 mL to about 10 mL, of the cleaning composition, preferably in liquid form, of the present invention diluted in water. The actual amount of cleaning composition used will be based on the judgment of the user and will typically depend upon factors such as the particular product formulation of the cleaning composition, including the concentration of active ingredients in the cleaning composition, the number of soiled dishes to be cleaned, the degree of soiling on the dishes, and the like. Generally, from about 0.01 mL to about 150 mL, preferably from about 3 mL to about 40 mL of a cleaning composition of the invention is combined with from about 2,000 mL to about 20,000 mL, more typically from about 5,000 mL to about 15,000 mL of water in a sink. The soiled dishware are immersed in the sink containing the diluted cleaning compositions then obtained, before contacting the soiled surface of the dishware with a cloth, sponge, or similar cleaning implement. The cloth, sponge, or similar cleaning implement may be immersed in the cleaning composition and water mixture prior to being contacted with the dishware, and is typically contacted with the dishware for a period of time ranged from about 1 to about 10 seconds, although the actual time will vary with each application and user. The contacting of cloth, sponge, or similar cleaning implement to the dishware is accompanied by a concurrent scrubbing of the dishware.

Alternatively, the composition herein can be applied in its neat form to the dish to be treated. By "in its neat form", it is meant herein that said composition is applied directly onto the surface to be treated, or onto a cleaning device or implement such as a brush, a sponge, a nonwoven material, or a woven material, without undergoing any significant dilution by the user (immediately) prior to application. "In its neat form", also includes slight dilutions, for instance, arising from the presence of water on the cleaning device, or the addition of water by the consumer to remove the remaining quantities of the composition from a bottle. Therefore, the composition in its neat form includes mixtures having the composition and water at ratios ranging from 50:50 to 100:0, preferably 70:30 to 100:0, more preferably 80:20 to 100:0, even more preferably 90: 10 to 100:0 depending on the user habits and the cleaning task.

Another aspect of the present invention is directed to use of the liquid hand dishwashing cleaning compositions, described herein, for providing good sudsing profile, including suds stabilization in the presence of soils and especially greasy-particulate soil, and good cleaning while providing good low temperature stability, at an increased bioderived surfactant content and biodegradability profile.

Processes for making alkyl alkanolamide sulfate anionic surfactants, and concentrated blends comprising them

The alkyl alkanolamide sulfate anionic surfactant can be made using any suitable process. For instance, a

nonionic stream can be provided, wherein the nonionic stream comprises at least one alkyl alkanolamide of formula (II):

R(CO)NXR'OH (II)

R, X, R' are as defined for formula (I).

The nonionic stream forms the feedstock for the sulfation step to form a sulfuric acid stream comprising at least one alkyl alkanolamide sulfuric acid. In the sulfation step, feedstock, which is the nonionic stream comprising the at least one alkyl alkanolamide, is sulfated to form a sulfuric acid stream comprising at least one alkyl alkanolamide sulfuric acid. Sulfation involves forming a carbon-oxygen-sulfur bond. The resultant alkyl alkanolamide sulfate in acid form is not hydrolytically stable. Unless neutralized, it decomposes to form sulfuric acid and other chemicals.

Similar processes for the sulfation of alcohols or alkoxylated alcohols into (alkoxylated) alkyl sulfuric acids has been described extensively. More details of such a sulfation step has been described in "Sulf(on)ation Technology in the Detergent Industry" (W. Herman de Groot, Springer-Science+Business Media, B.V., 1991, ISBN 978-90-481-4088-6).

Various reagents can be used for the sulfation step, with sulfur trioxide ($SO_3$) being particularly preferred, at least partially due to its low cost. $SO_3$ is an aggressive electrophilic reagent that rapidly reacts with any organic compound containing an electron donor group. The resultant reaction is highly exothermic. Effective cooling of the reaction mass is essential because high temperatures promote side reactions that produce undesirable by-products. Also, precise control of the molar ratio of $SO_3$ to alkyl alkanolamide (and alkyl alcohol, if present) is essential because any excess $SO_3$, due to its reactive nature, contributes to side reactions and by-product formation. Therefore, commercial scale sulfation reactions require special equipment and instrumentation that allows tight control of the mole ratio of $SO_3$ to the alkyl alkanolamide (and alkyl alcohol, if present) and rapid removal of the heat of reaction.

The problem of $SO_3$ reactivity has typically been solved by diluting and/or complexing the $SO_3$ to moderate the rate of reaction. Commercial diluting or complexing agents include ammonia (sulfamic acid), hydrochloric acid (chlorosulfuric acid), and dry air (air/$SO_3$ film sulfation). Control of the ratio of $SO_3$ to alkyl alkanolamide (and alkyl alcohol, if present) can be used to achieve improved product quality with use of any of these reagents.

Air/$SO_3$ film sulfation processes are typically carried out using a film reactor, such as an annular falling film reactor, such as a "Chemithon" reactor, or a multi-tube film reactor, such as the "Ballestra" reactor. In such processes, the $SO_3$ is first diluted with dry air. Such air/$SO_3$ sulfation processes are direct processes in which $SO_3$ gas is diluted with very dry air and reacted directly with the feedstock. The reaction of gaseous $SO_3$ with the alkyl alkanolamide (and alkyl alcohol, if present) is rapid and stoichiometric. Such processes are complicated by the possibility of side reactions, However, with tight process control, very high purity sulfate surfactants can be achieved.

In air/$SO_3$ film sulfation processes, the sulfation step is typically carried out in a liquid-gas interface reactor, preferably a falling film reactor. Suitable falling film reactors include annular-gap falling film ("Chemithon") reactors, (multi) tubular ("Ballestra") reactors, and the like.

The alkyl alkanolamide (and alkyl alcohol, if present) is converted through reaction with the $SO_3$ within the falling film reactor into the sulfuric acid. Considering the exothermic nature of the sulfonation reaction, consequent cooling is again required after which the air/gas is separated from the liquid stream. Ideally the reaction mixture is maintained at the outlet of the reactor at a temperature of from 15 °C to 50 °C, preferably from 30 °C and 40°C.

If needed, a degassing step can be done.

The nonionic stream fed into the sulfation reaction preferably comprises less than 0.1%, preferably less than 0.05%, more preferably is free of water.

The nonionic stream further comprises at least one alkyl alcohol and/or at least one alkoxylated alkyl alcohol, such that the nonionic stream comprises the at least one alkyl alcohol and/or at least one alkoxylated alkyl alcohol and at least one alkyl alkanolamide of formula (II). Such processes can be used to make a concentrated surfactant blend, wherein the concentrated surfactant blend comprises alkyl sulfate anionic surfactant and the alkyl alkanolamide sulfate anionic surfactant of formula (I). The at least one alkyl alcohol has an average degree of alkoxylation of less than 0.1, and is preferably free of alkoxykation.

During the subsequent sulfation step, the at least one alkyl alcohol and/or at least one alkoxylated alkyl alcohol and the at least one alkyl alkanolamide of formula (II) in the nonionic stream is sulfated to form a sulfuric acid stream comprising at least one alkyl sulfuric acid and/or at least one alkoxylated alkyl sulfuric acid and at least one alkyl alkanolamide sulfuric acid. The sulfuric acid stream is then combined with the neutralising stream during the neutralisation step, in order to neutralise the at least one alkyl sulfuric acid and/or the at least one alkoxylated alkyl sulfuric acid and at least one alkyl alkanolamide sulfuric acid.

The neutralizing agent in the neutralising stream can be an alkali metal hydroxide, or ammonium hydroxide, ethanolamine, or isopropanolamine, more preferably sodium hydroxide, potassium hydroxide, magnesium hydroxide, or ethanolamine, most preferably sodium hydroxide.

A buffering surfactant can be added before or during the neutralisation step to provide a more hydrolytically stable sulfated anionic surfactant. Such sulfated anionic surfactants can be kept at a lower pH. Suitable buffering surfactants are

amphoteric surfactant, zwitterionic surfactant, or mixtures thereof. Suitable buffering surfactants can be selected from the group consisting of: amine oxide surfactant, betaine surfactant, and mixtures thereof, preferably amine oxide surfactant, more preferably C10-16 dimethyl amine oxide surfactant. C12-14 dimethyl amine oxide (lauryl dimethyl amine oxide) is particularly preferred. Such buffering surfactants also contribute to the performance of the resultant liquid hand dishwashing detergent. In addition, the buffering surfactant results in the concentrated surfactant blends, formed by the processes described herein, having reduced viscosity, especially when amine oxide surfactant is used as the buffering surfactant.

The buffering surfactant is preferably added during the neutralization step, either as a separate buffer stream, but is preferably added as part of the neutralisation stream. Alternatively, the buffering surfactant can be added before the neutralisation step. The alkyl alkanolamide sulfuric acid (and alkyl sulfuric acid and/or alkoxylated alkyl sulfuric acid, if present) and the buffering surfactant can be combined in a weight ratio of the combined alkyl sulfuric acid and/or alkoxylated alkyl sulfuric acid and alkyl alkanolamide sulfuric acid to the buffering surfactant of from 10:1 to 1:1, preferably from 8:1 to 2:1, more preferably from 6:1 to 3:1.

METHODS

Viscosity measurement

The viscosity is measured using a controlled stress rheometer (such as an HAAKE MARS from Thermo Scientific, or equivalent), using a 60 mm 1° cone and a gap size of 52 microns at 20°C. After temperature equilibration for 2 minutes, the sample is sheared at a shear rate of 10 $s^{-1}$ for 30 seconds. The reported viscosity of the liquid hand dishwashing detergent compositions is defined as the average shear stress between 15 seconds and 30 seconds shearing divided by the applied shear rate of 10 $s^{-1}$ at 20°C.

Suds mileage in presence of grease-particulate soil

The objective of the Suds Mileage Test is to compare the evolution over time of suds volume generated for different test formulations at specified water hardness, solution temperatures and formulation concentrations, while under the influence of periodic grease-particulate soil injections. Data are compared and expressed versus a reference composition as a suds mileage index (reference composition has suds mileage index of 100). The steps of the method are as follows:

1. 0.12 wt% of the test composition is dispensed through a plastic pipette at a flow rate of 0.67 mL/ sec at a height of 37 cm above the bottom surface of a sink (dimension: 300 mm diameter and 288 mm height) into a water stream having a water hardness of 2.67 mmol/L equivalence of Ca (15 dH) and temperature of 42°C, that is filling up the sink to 4 L at a constant pressure of 4 bar.
2. An initial suds volume generated (measured as average foam volume X above the liquid in the sink (expressed in $cm^3$) is recorded immediately after end of filling.
3. A fixed amount (6 mL) of a soil with one of the defined compositions below is immediately injected into the middle of the sink.
4. The resultant solution is mixed with a metal blade (10 cm × 5 cm) positioned in the middle of the sink at the air liquid interface under an angle of 45° rotating at 85 RPM for 20 revolutions.
5. Another measurement of the total suds volume is recorded immediately after end of blade rotation.
6. Steps 3-5 are repeated until the measured total suds volume reaches a level of 400 $cm^3$ or less. The amount of added soil that is needed to get to the 400 $cm^3$ level is considered as the suds mileage for the test composition.
7. Each test composition is tested 4 times per testing condition (i.e., water temperature, composition concentration, water hardness, soil type).
8. The average suds mileage is calculated as the average of the 4 replicates for each sample for a defined test condition.
9. The Suds Mileage Index is calculated by comparing the average mileage of a test composition sample versus a reference composition sample. The calculation is as follows:

$$\text{Suds Mileage Index} = \frac{\text{Average number of soil additions of test composition}}{\text{Average number of soil additions of reference composition}} \text{ X } 100$$

The grease-particulate soil composition used in the test is produced through standard mixing of the components described in Table 1.

Table 1: grease-particulate Soil

| Ingredient | Weight % |
|---|---|
| Zwan® Carbonades | 22.67 |
| Beaten Eggs | 4.78 |
| Smash® Instant Mash Potato | 9.26 |
| McDougall's® Sponge Mix | 3.30 |
| Milk UHT Full Cream | 22.22 |
| Bisto® Gravy Granules | 1.30 |
| Mazola® Pure Corn Oil | 9.29 |
| Demineralized water | 26.32 |
| Sodium Benzoate | 0.42 |
| Potassium Sorbate | 0.42 |

C) Grease cleaning

The grease cleaning performance test method is used to measure the relative grease removal efficacy from compositions across different dilution concentrations in water, at the specified hardness and temperature (0.5 wt%, 1.0 wt%, 1.5 wt%, 5.0 wt%, 10 wt% concentrations of the liquid detergent composition in water of hardness 0.356 mmol $CaCO_3$ equivalence (2 dH), 2.67 mmol $CaCO_3$ equivalence (15 dH), and 5.34 mmol $CaCO_3$ equivalence (30 dH), at a temperature of 35 °C) versus a reference composition.

Homogenised lard (soil #44069 supplied by Warwick Equest Ltd. UK) is preheated to 50 °C, mixed with a violet dye for improved visibility and kept in an oven for 2 hours. A plastic syringe is then filled with 50 ml of the coloured homogenised lard, ensuring there is no air in the syringe, before being left in an oven at 60 °C. The syringe is then left for at least 24 hours at 22 °C to ensure the coloured homogenised lard has solidified.

The coloured homogenised lard is then heated to 55 °C. A Liquidyn® P-Jet Series jetting valve (article # 7825004, supplied by Nordson, Westlake, Ohio 44145-4551) using a valve pressure of 5 bar, fluid pressure of 3 bar, and a valve opening time of 2 ms, is used to inject the heated coloured homogenised lard onto 3 layers of polypropylene non-woven substrate (14 cm by 9 cm, 60 g/m2 sms nonwoven produced by Avgol) placed side-by-side, to print 96 circular stains (each 5 mm diameter) into the nonwoven, to create stain circles of roughly 5mm diameter, each comprising circa 5 mg of the coloured homogenised lard. The stained fabric is then dried for 24 hours at 21 °C.

The fabric stains are then cut from the treated nonwoven using a 5 mm diameter cutting mould and a hydraulic ATOM 1 press plate. Each cut fabric stain is then placed at the bottom of a well of a 96 well deep-well microtiter plate (96-well plate, polystyrene, U-bottom, round well shape, 1,1 ml, part number 391-0159, supplied by VWR).

The stain intensity is measured before and after treatment through imaging using a DigiEye® (supplied by Verivide UK, Z02791) under D65 diffuse light) with a Nikon D7000 camera, which determines the average grease stain intensity before and after washing by converting the RGB values to on L a b measurements.

Wash solutions are prepared at the target composition concentrations, water hardness and temperature. The wash solution was then dosed (950 $\mu$L wash solution per well) into each well using a 96-channel pipetting head, for a contact time of 10 minutes of the stained fabric with the swash solution. The wash solution was then removed using the 96-channel pipetting head. 4 rinse cycles were applied using the 96-channel pipetting head, each rinse cycle consisting of 950 $\mu$L of water at 0.356 mmol/l Ca calcium equivalence water hardness and 35 °C, applied for 2 minutes, before the rinse water was removed using the pipetting head. The substrates were then dried for 24 hours at 30 °C.

The stain intensity after the treatment was then remeasured through imaging using the DigiEye® (supplied by Verivide UK, Z02791) and imaging software to determine the average grease stain intensity after washing.

The percentage stain removal (% SR) is then calculated for each wash solution concentration as:

$$\%\ SR = \frac{\%\ \text{stain area before washing} - \%\ \text{stain area after washing}}{\%\ \text{stain area before washing}} \times 100$$

The % SR is averaged over the 8 replicates for each test condition (wash concentration and water hardness) and consequently plotted against the different wash concentrations. The area under the resulting curve was calculated and indexed versus the area under the curve for the reference product. The % SRI Index is calculated by comparing the area under the curve for the test composition versus the area under the curve for the reference composition sample:

$$\% \text{ SRI} = \frac{\text{area under the curve for the test composition}}{\text{area under the curve for the ref. composition}} \times 100$$

EXAMPLES

The suds mileage and grease cleaning performance of compositions of the present invention, comprising alkyl alkanolamide sulfate surfactant as part of the anionic surfactant, and comparative compositions only comprising alkyl sulfate anionic surfactant or alkyl ethoxylated sulfate anionic surfactant as the anionic surfactant was compared, using the test methods described herein.

The lauryl (C12-C14) monoethanolamide sulfate was obtained through the sulfation of lauryl monoethanolamide (CAS: 142-78-9). The lauryl monoisopropanolamide sulfate was obtained through the sulfation of lauryl (C12-C14) monoisopropanolamide (CAS:142-54-1). The alkyl sulfate anionic surfactant and ethoxylated alkyl sulfate anionic surfactant were obtained through sulfation of the respective non-ethoxylated and ethoxylated alcohols. The sulfations were all carried out on a pilot-scale sulfation unit.

The following inventive and comparative compositions were prepared through mixing of the individual starting ingredients. The compositions comprised a blend of linear alkyl alkanolamide sulfate in combination with branched alkyl sulfate as the anionic surfactant for the inventive compositions, and a blend of linear ethoxylated sulfate or linear alkyl sulfate in combination with branched alkyl sulfate as the anionic surfactant for the comparative examples.

Table 2: Inventive compositions and comparative compositions:

| | Ex 1 | Ex 2 | Ex A* | Ex B* |
|---|---|---|---|---|
| Lauroyl Amido Ethyl Sulfate [3] | 6.01 | - | - | - |
| Lauroyl Amido Isopropyl Sulfate[4] | - | 6.01 | - | - |
| C12-C14 linear alkyl ethoxylated (EO3.0) sulfate[2] | - | - | 8.12 | - |
| C12-C14 linear alkyl sulfate | - | - | - | 6.01 |
| C12-C13 branched alkyl sulfate' | 16.37 | 16.37 | 16.37 | 16.37 |
| C12-14 dimethyl amine oxide[5] | 7.41 | 7.41 | 7.41 | 7.41 |
| Ethanol | 4.28 | 4.28 | 4.28 | 4.28 |
| Polypropylene glycol (mw 2000) | 0.63 | 0.63 | 0.63 | 0.63 |
| Alkoxylated Polyethyleneimine[6] | 0.53 | 0.53 | 0.53 | 0.53 |
| NaCl | 0.74 | 0.74 | 0.74 | 0.74 |
| Water + misc.[7] | to 100% | to 100% | to 100% | to 100% |
| Trimmed to pH (using 10% aqueous solution of NaOH or HCl at 20°C) | to 9.0 | to 9.0 | to 9.0 | to 9.0 |
| | | | | |

* Comparative
[1] C12-C13 branched alkyl sulfate anionic surfactant (54% branching), supplied by Procter & Gamble and derived from Safol® 23 alkyl alcohol, supplied by Sasol
[2] Tensagex® EOC970, supplied by KLK Tensachem
[3] obtained through the sulfation of lauryl (C12-C14) monoethanolamide (CAS: 142-78-9), sold under the trade name Ninol® LMP, by Stepan
[4] obtained through the sulfation of lauryl (C12-C14) monoisopropanolamide (CAS: 142-54-1), sold under the trade name ColaMid® LMPA, by Colonial Chemical
[5] supplied by Procter & Gamble
[6] Polyethyleneimine with PEI backbone MW of 600 and 24EO and 16PO units per alkoxylation chain, supplied by BASF
[7] Perfume, dye, preservative

Suds mileage, especially in the presence of greasy-particulate soil, is increased for compositions comprising alkyl ethoxylated surfactant, in comparison to equivalent compositions where all of the anionic surfactant is non-alkoxylated. In contrast, greasy soil removal is improved for compositions comprising non-alkoxylated alkyl sulfate anionic surfactant, in comparison to compositions comprising alkyl ethoxylated sulfate anionic surfactant. As such, the suds mileage of the inventive compositions, comprising alkyl alkanolamide sulfate anionic surfactant was evaluated using a composition comprising alkyl ethoxylated anionic surfactant as the reference.

Since linear (lauroyl) alkyl alkanolamide sulfate anionic surfactant was used in the inventive compositions, C12-C14 linear alkyl ethoxylated alkyl sulfate anionic surfactant was used in the reference composition used for evaluating suds mileage.

Since linear (lauroyl) alkyl alkanolamide sulfate anionic surfactant was used in the inventive compositions, C12-C14 linear alkyl (non-ethoxylated) alkyl sulfate anionic surfactant was used in the reference composition used for evaluating grease cleaning.

The suds mileage index in hard water having a hardness of 2.67 mmol/L equivalence of Ca (15 dH) was evaluated for both inventive examples 1 and 2, using comparative example A as the reference. The composition of comparative example A comprised a combination of linear alkyl ethoxylated sulfate and branched alkyl sulfate anionic surfactant, with an anionic surfactant level which was 2 wt% higher than that of inventive examples 1 and 2.

Table 3: Suds mileage index of inventive compositions 1 and 2 in hard water of hardness 2.67 mmol/L equivalence of Ca (15 dH), using comparative composition A as the reference (100):

| Suds mileage index vs comparative example A | Ex 1 | Ex 2 |
|---|---|---|
| @ hardness 2.67 mmol/L equivalence of Ca (15 dH) | 108 | 104 |

From the results above, it can be seen that when the linear ethoxylated alkyl sulfate anionic surfactant is replaced with the alkyl alkanolamide sulfate anionic surfactant, suds mileage in hard water was improved, even though the linear ethoxylated alkyl sulfate anionic surfactant was replaced by a lower amount of the alkyl alkanolamide sulfate anionic surfactant.

The grease cleaning performance of the compositions of inventive examples 1 and 2 were evaluated, using comparative example B as the reference (comprising linear non-ethoxylated alkyl sulfate anionic surfactant).

Table 4: Grease cleaning performance of inventive compositions 1 and 2 at different water hardness, using comparative composition B as the reference (100):

| Grease cleaning index vs comparative example B | Ex 1 | Ex 2 |
|---|---|---|
| @ hardness 0.356 mmol/L equivalence of Ca (2 dH) | 159 | 129 |
| @ hardness 2.67 mmol/L equivalence of Ca (15 dH) | 107 | 105 |
| @ hardness 5.34 mmol/L equivalence of Ca (30 dH) | 106 | 107 |

From the results above, it can be seen that when the linear alkyl sulfate is replaced with an alkyl alkanolamide sulfate anionic surfactant of use in the present invention, grease cleaning was improved for all water hardness used.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

**1.** A liquid hand dishwashing detergent composition comprising from 5.0% to 50% by weight of the liquid hand dishwashing detergent composition of a surfactant system, wherein the surfactant system comprises anionic surfactant, wherein the anionic surfactant comprises:

a. an alkyl alkanolamide sulfate anionic surfactant, wherein the alkyl alkanolamide sulfate anionic surfactant has the formula:

$$R(CO)NXR'O\text{-}SO_3^- M^+ \quad (I),$$

wherein:

R is an alkyl chain comprising a number average of from 7 to 17 carbon atoms;
R' is an alkyl chain comprising a number average of from 1 to 3 carbon atoms;
X is H or C1 to C3 alkyl; and
$M^+$ is a counterion; and

b. alkyl sulfate anionic surfactant, wherein the alkyl sulfate anionic surfactant has an average degree of alkoxylation of less than 0.1.

**2.** The composition according to claim 1, wherein the composition comprises from 6.0% to 40%, preferably from 15% to 35%, by weight of the total composition of the surfactant system.

**3.** The composition according to any of the preceding claims, wherein the surfactant system comprises at least 40%, preferably from 60% to 90%, more preferably from 65% to 85% by weight of the surfactant system of the anionic surfactant.

**4.** The composition according to any of the preceding claims, wherein the anionic surfactant comprises at least 70%, more preferably at least 85%, most preferably 100% by weight of the anionic surfactant of the alkyl sulfate anionic surfactant and the alkyl alkanolamide sulfate anionic surfactant.

**5.** The composition according to any of the preceding claims, wherein in the alkyl alkanolamide sulfate anionic surfactant of formula (I):

R is an alkyl chain comprising a number average of from 9 to 13, preferably from 11 to 13 carbon atoms, most preferably R is a blend of C11 and C13 alkyl chains;
R' is an alkyl chain comprising a number average of from 2 to 3 carbon atoms, more preferably wherein R' is selected from the group consisting of: $-(CH_2)_2-$, $-CH_2CH(CH_3)-$, $-CH(CH_3)CH_2-$;
X is H or C1 alkyl, preferably H or methyl,
$M^+$ is an alkali metal counterion or ammonium, ethanolamine, or isopropanolamine, more preferably $Na^+$, or $K^+$, $Mg^{2+}$, or ethanolamine, most preferably $Na^+$.

**6.** The composition according to claim 5, wherein in the alkyl alkanolamide sulfate anionic surfactant, the alkyl chain R has a mole percentage of C11 to C13 chains to total alkyl chains of at least 60%, preferably at least 70%, more preferably at least 80%, most preferably at least 90%.

**7.** The composition according to any of the preceding claims, wherein the alkyl alkanolamide sulfate anionic surfactant comprises C12 alkyl alkanolamide sulfate anionic surfactant, C14 alkyl alkanolamide sulfate anionic surfactant, and mixtures thereof, with a blend of C12 alkyl alkanolamide sulfate anionic surfactant and C14 alkyl alkanolamide sulfate anionic surfactant being preferred,
wherein:

the C12 alkyl alkanolamide sulfate anionic surfactant is preferably selected from the group consisting of: N-(2-hydroxyethyl)dodecanamide sulfate, N-(2-hydroxypropyl)dodecanamide sulfate, N-(2-hydroxyethyl)-N-methyldodecanamide sulfate, N-(1-hydroxypropan-2-yl)dodecanamide sulfate, and mixtures thereof, preferably N-(2-hydroxyethyl)dodecanamide sulfate, N-(2-hydroxypropyl)dodecanamide sulfate, N-(2-hydroxyethyl)-N-methyldodecanamide sulfate, and mixtures thereof; and
the C14 alkyl alkanolamide sulfate anionic surfactant is preferably selected from the group consisting of: N-(2-hydroxyethyl)tetradecanamide sulfate, N-(2-hydroxypropyl)tetradecanamide sulfate, N-(2-hydroxyethyl)-N-methyltetradecanamide sulfate, N-(1-hydroxypropan-2-yl) tetradecanamide sulfate, and mixtures thereof, preferably N-(2-hydroxyethyl) tetradecanamide sulfate, N-(2-hydroxypropyl) tetradecanamide sulfate, N-(2-hydroxyethyl)-N-methyltetradecanamide sulfate, and mixtures thereof.

**8.** The composition according to any preceding claim, wherein the alkyl sulfate anionic surfactant has a number average alkyl chain length of from 8 to 18, preferably from 10 to 14, more preferably from 12 to 14, most preferably from 12 to 13 carbon atoms.

**9.** The composition according to any preceding claim, wherein the alkyl sulfate anionic surfactant is free of alkoxylation.

**10.** The composition according to any preceding claim, wherein the alkyl sulfate anionic surfactant has an average degree of branching of at least 15%, preferably from 20% to 60%, more preferably from 30% to 50%.

**11.** The composition according to any preceding claim, wherein the alkyl sulfate anionic surfactant and the alkyl alkanolamide sulfate anionic surfactant are present at a weight ratio of from 10:1 to 1:2, preferably from 7:1 to 1:1, and most preferably from 5:1 to 2:1.

**12.** The composition according to any preceding claim, wherein the surfactant system further comprises a co-surfactant selected from the group consisting of: amphoteric co-surfactant, zwitterionic co-surfactant, and mixtures thereof.

**13.** The composition according to claim 12, wherein the anionic surfactant and the co-surfactant are present in a weight ratio of from 1:1 to 8:1, preferably from 2:1 to 5:1, more preferably from 2.5:1 to 4:1,
wherein:

the amphoteric surfactant is preferably an amine oxide surfactant, more preferably wherein the amine oxide surfactant is selected from the group consisting of: alkyl dimethyl amine oxide, alkyl amido propyl dimethyl amine oxide, and mixtures thereof, most preferably alkyl dimethyl amine oxide; and
the zwitterionic surfactant is preferably a betaine surfactant, more preferably a betaine surfactant selected from the group consisting of alkyl betaines, alkylamidoalkylbetaine, amidazoliniumbetaine, sulfobetaine (INCI Sultaines), phosphobetaine, and mixtures thereof, most preferably cocoamidopropylbetaine.

**14.** A process for making a concentrated surfactant blend comprising the alkyl alkanolamide sulfate anionic surfactant having the formula:

R(CO)NXR'O-SO3- M+ (I),

and alkyl sulfate anionic surfactant, wherein the alkyl sulfate anionic surfactant has an average degree of alkoxylation of less than 0.1;
wherein the process comprises the following steps:

a. providing a nonionic stream, wherein the nonionic stream comprises:

at least one alkyl alkanolamide of formula (II):

R(CO)NXR'OH (II),

and
at least one alkyl alcohol, wherein the at least one alkyl alcohol has an average degree of alkoxylation of less than 0.1;

b. a sulfation step, during which the at least one alkyl alkanolamide of formula (II) and at least one alkyl alcohol in the nonionic stream is sulfated to form a sulfuric acid stream comprising at least one alkyl alkanolamide sulfuric acid and at least one alkyl sulfuring acid;
c. providing a neutralising stream comprising at least one neutralising agent;
d. a neutralisation step, during which the sulfuric acid stream and the neutralising stream are combined, in order to neutralise the at least one alkyl alkanolamide sulfuric acid and at least one alkyl sulfuric acid.

Europäisches Patentamt
European Patent Office
Office européen des brevets

# EUROPEAN SEARCH REPORT

Application Number
EP 24 22 2297

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 0 780 367 A2 (HENKEL KGAA [DE]) 25 June 1997 (1997-06-25)<br>* page 2, line 29 - page 3, line 12 *<br>* page 4, lines 10-14 *<br>* examples; table 1 *<br>* claims * | 1-14 | INV.<br>C11D11/04<br>C07C303/24<br>C11D1/37<br><br>ADD.<br>C11D1/12<br>C11D1/14<br>C11D1/29 |
| X,D | US 4 116 986 A (BISTLINE JR RAYMOND G ET AL) 26 September 1978 (1978-09-26)<br>* column 1, line 54 - column 2, line 2 *<br>* examples *<br>* claims * | 1,3-5, 9-11,14 | |
| X,D | JP H10 330783 A (LION CORP) 15 December 1998 (1998-12-15)<br>* paragraph [0006] *<br>* examples *<br>* claims * | 1-14 | |
| X,D | KR 2012 0060485 A (AE KYUNG IND CO LTD [KR]) 12 June 2012 (2012-06-12)<br>* paragraphs [0007], [0014] *<br>* examples; table 4 *<br>* claims * | 1-14 | TECHNICAL FIELDS SEARCHED (IPC)<br>C11D<br>C07C |
| X,D | US 2 353 081 A (ROBINSON EDWIN A ET AL) 4 July 1944 (1944-07-04)<br>* page 1, right-hand column, lines 4-49 *<br>* page 2, left-hand column, lines 24-67 *<br>* examples *<br>* claims * | 1-14 | |
| A | EP 0 070 075 A2 (PROCTER & GAMBLE [US]) 19 January 1983 (1983-01-19)<br>* page 1, line 26 - page 2, line 34 *<br>* page 14, lines 4-17 *<br>* examples *<br>* claims * | 1-14 | |

-/--

The present search report has been drawn up for all claims

1

| Place of search | Date of completion of the search | Examiner |
|---|---|---|
| The Hague | 12 June 2025 | Bertran Nadal, Josep |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 24 22 2297

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GB 709 557 A (WOLFGANG BENJAMIN REINISCH ET AL.) 26 May 1954 (1954-05-26)<br>* page 2, lines 1-31, 79-93 *<br>* page 3, lines 119-126 *<br>* example IV *<br>* claims *<br>----- | 1-4,14 | |
| X | US 2 581 677 A (MACHLIS SAMUEL ET AL) 8 January 1952 (1952-01-08)<br>* column 2, lines 12-30 *<br>* column 2, line 50 - column 3, line 7 *<br>* examples 1, 2 *<br>* claims *<br>----- | 1-4,8,9,11 | |

TECHNICAL FIELDS SEARCHED (IPC)

The present search report has been drawn up for all claims

1

| Place of search | Date of completion of the search | Examiner |
|---|---|---|
| The Hague | 12 June 2025 | Bertran Nadal, Josep |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 24 22 2297

This annex lists the patent family members relating to the patent documents cited in the above-mentioned European search report. The members are as contained in the European Patent Office EDP file on
The European Patent Office is in no way liable for these particulars which are merely given for the purpose of information.

12-06-2025

| Patent document cited in search report | Publication date | Patent family member(s) | Publication date |
|---|---|---|---|
| EP 0780367 A2 | 25-06-1997 | DE 19547458 A1 | 19-06-1997 |
| | | EP 0780367 A2 | 25-06-1997 |
| | | ES 2140013 T3 | 16-02-2000 |
| US 4116986 A | 26-09-1978 | CA 1094092 A | 20-01-1981 |
| | | US 4116986 A | 26-09-1978 |
| JP H10330783 A | 15-12-1998 | NONE | |
| KR 20120060485 A | 12-06-2012 | NONE | |
| US 2353081 A | 04-07-1944 | NONE | |
| EP 0070075 A2 | 19-01-1983 | NONE | |
| GB 709557 A | 26-05-1954 | NONE | |
| US 2581677 A | 08-01-1952 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0780367 A
- US 4116986 A
- JP H10330783 A
- KR 20120060485 A
- US 2353081 A
- US 2843550 A
- EP 4253510 A
- WO 2007135645 A
- WO 2019108293 A1


### Non-patent literature cited in the description


- Synthesis and properties of sulfated alkanolamides. *Journal of the American Oil Chemists' Society*, 1970, vol. 47, 91-93
- Synthesis and Properties of N-Alkyl Amide Sulfates. *Langmuir*, 1999, vol. 15 (20), 6664-6670
- **W. HERMAN DE GROOT**. Sulf(on)ation Technology in the Detergent Industry. Springer-Science+Business Media, B.V, 1991
- *CHEMICAL ABSTRACTS*, 142-78-9
- *CHEMICAL ABSTRACTS*, 142-54-1